(19) Europäisches Patentamt
European Patent Office
Office européen des brevets

(11) Veröffentlichungsnummer: **0 466 009 A1**

## (12) EUROPÄISCHE PATENTANMELDUNG

(21) Anmeldenummer: **91110998.1**

(22) Anmeldetag: **03.07.91**

(51) Int. Cl.5: **G01N 1/02**, C12M 1/30, A61B 10/00

(30) Priorität: **10.07.90 CH 2304/90**

(43) Veröffentlichungstag der Anmeldung: **15.01.92 Patentblatt 92/03**

(84) Benannte Vertragsstaaten: **CH DE FR GB IT LI NL**

(71) Anmelder: **F. HOFFMANN-LA ROCHE AG** Postfach 3255 CH-4002 Basel(CH)

(72) Erfinder: **Burkardt, Hans-Joachim** **Burgackerweg 13** **W-7889 Grenzach-Wyhlen(DE)**
Erfinder: **Weber, Wilhelm** **Roettler Ring 24** **W-7889 Grenzach-Wyhlen(DE)**

(74) Vertreter: **Ventocilla, Abraham et al** **Grenzacherstrasse 124 Postfach 3255** **CH-4002 Basel(CH)**

(54) **Transportsystem für den Versand von biologischen Proben.**

(57) Zur Erzielung der an einem Transportsystem für mikrobiologischen Proben gestellten Anforderungen, u.a. Erhaltung der Lebensfähigkeit der in der Probe enthaltenen Keime und Schutz der Umwelt vor Auslaufen der Proben ist das Transportsystem dadurch gekennzeichnet, dass es folgende Komponenten enthält:

(a) einen ersten Behälter (11), der mit einem abnehmbaren Verschluss (12) versehen ist und ein Medium (13) zur Aufbewahrung der Probe enthält, und

(b) einen zweiten Behälter (14), der mit einer abnehmbaren Verschlusskappe (15) versehen ist, die eine längliche Kammer (21) hat, in die ein Ende eines Stäbchens (16) angeordnet ist und hineingeschoben werden kann, welches Stäbchen an dessen andererem Ende einen Abstrichtupfer (17) trägt,

wobei die Abmessungen des ersten und des zweiten Behälters, der Kammer (21) in der Verschlusskappe (15) und des Stäbchens (16) mit dem Abstrichtupfer (17) so gewählt werden, dass der erste Behälter (11) und ein darin im Medium (13) eingeschobenes Stäbchen (16) mit Abstrichtupfer (17) in dem mit der Verschlusskappe (15) verschlossenen zweiten Behälter (14) enthalten sein können.

Fig. 3

Die Erfindung betrifft ein Transportsystem für den Versand von biologischen Proben, insbesondere für den Versand von Proben die in der mikrobiologischen Diagnostik untersucht werden.

Mikrobiologische Proben, z.B. Abstriche, werden von Patienten entnommen. Oft können solche Proben aus räumlichen oder Zeitlichen Gründen nicht sofort weiterverarbeitet werden, und müssen zu einem Untersuchungslabor geschickt werden. Dafür ist es erwünscht, die Proben in ein Transportsystem überführen zu könen, durch welches folgende Bedingungen sicher gestellt werden:

- Die Lebensfähigkeit der in der mikrobiologischen Proben enthaltenden Keime muss erhalten bleiben. Dafür müssen diese insbesondere vor Austrocknung, vor Luftsauerstoff und vor eigenen toxischen Stoffwechselprodukten geschützt werden.
- Die ursprünglichen biologischen Zusammensetzung der Probe muss erhalten bleiben. Z.B. ist bei Mischkulturen ein Ueberwachsen einer Spezies durch die andere zu verhindern.
- Die Umwelt muss während des Transports vor Auslaufen der Proben geschützt werden. Dafür ist es besonders wichtig, den Probenbehälter vor Bruch zu schützen.
- Zur Weiterverarbeitung der Proben muss das Transportsystem eine sichere und saubere Probeentnahme im Untersuchungslabor ermöglichen.

Es ist ein Transportsystem für Mikroorganismen bekannt, das von der Firma Merck, Darmstadt, BRD, mit der Bezeichnung Transgerm (Warenzeichen) kommerzialisiert wird. Dieses System besteht im wesentlichen aus einem Glasröhrchen, das mit einem Schraubverschluss versehen ist und ein Medium für den Transport einer Probe enthält, und aus einem steril verpackten Abstrichtupfer. Bei der Verwendung dieses Systems muss das Stäbchen des Abstrichtupfers nach Durchführung des Abstrichs abgebrochen werden, damit der Abstrichtupfer eine passende Länge bekommt, um in das Glasröhrchen eingeschoben und im verschlossenen Glasröhrchen z.B. per Post verschickt werden zu können. Zur Erleichterung der Handhabung des Abstrichtupfers hat der Schraubverschluss des Glasröhrchens eine längliche, zylindriche Kammer, in die das freie Ende des Stäbchens des Abstrichtupfers - nach dem Abbrechen des Stäbchens - eingeschoben werden kann. Der Hauptnachteil dieses bekannten Transportsystems ist, das es keine Bruchsicherung für das Glasröhrchen vorsieht. Die Notwendigkeit, jedesmal das Stäbchen des Abstrichtupfers abbrechen zu müssen, macht die Benutzung dieses Systems relativ umständlich.

Der Erfindung liegt die Aufgabe zugrunde, ein Transportsystem zur Verfügung zu stellen, das sämtliche oben erwähnten Anforderungen erfüllt.

Erfindungsgemäss wird diese Aufgabe mit einem Transportsystem gelöst, das dadurch gekennzeichnet ist, dass es folgende Komponenten enthält:

(a) einen ersten Behälter, der mit einem abnehmbaren Verschluss versehen ist und ein Medium zur Aufbewahrung der Probe enthält, und

(b) einen zweiten Behälter, der mit einer abnehmbaren Verschlusskappe versehen ist, die eine längliche Kammer hat, in die ein Ende eines Stäbchens angeordnet ist und hineingeschoben werden kann, welches Stäbchen an dessen andererem Ende einen Abstrichtupfer trägt,

wobei die Abmessungen des ersten und des zweiten Behälters, der Kammer in der Verschlusskappe und des Stäbchens mit dem Abstrichtupfer so gewählt werden, dass der erste Behälter und ein darin im Medium eingeschobenes Stäbchen mit Abstrichtupfer in dem mit der Verschlusskappe verschlossenen zweiten Behälter enthalten sein können.

Mit dem erfindungsgemässen Transportsystem werden insbesondere folgende Vorteile erzielt:

- Das Stäbchen der Abstrichtupfer muss nach deren Einführung in das im ersten Behälter enthaltenen Transportmedium nicht abgebrochen werden (was bei einigen bisher bekannten Transportsystemen erforderlich ist), sondern gleitet ohne Manipulation in die entsprechend gestaltete Verschlusskappe des zweiten Behälters.
- Der zweite Behälter, dient sowohl als Träger und Verpackung des Abstrichtupfers (dessen Stäbchen in seiner Verschlusskappe angeordnet ist) vor dessen Gebrauch, wie auch als Schutzhülse für den ersten Behälter während des Probentransports.
- Die Benutzung des Abstrichtupfers wird durch den Umstand erleichtert, dass dessen Stäbchen vom Anfang an und über die ganze Benutzungsdauer des Systems, d.h. bei der Durchführung des Abstrichs, während des Transports zum Untersuchungslabor, bei der Entnahme des Abstrichtupfers aus dem Transportsystem und bei der Benutzung des Abstrichtupfers im Untersuchungslabor, in der Verschlusskappe des zweiten Behälters eingesteckt bleiben kann.

In einer bevorzugten Ausführungsform ist die Verschlusskappe des zweiten Behälters so gestaltet dass wenn der verschlossene zweite Behälter den ersten Behälter samt einen darin im Medium eingeschobenen Stäbchen mit Abstrichtupfer enthält, die Verschlusskappe gleichzeitig den ersten und den zweiten Behälter verschliesst.

Die längliche Kammer in der Verschlusskappe des zweiten Behälters ist annähernd zylindrisch und hat vorzugsweise einen Durchmesser, der in der Richtung vom offenen Ende der Kappe zum geschlossenen Ende derselben leicht abnimmt.

Eine bevorzugte Ausführungsform des erfindungsgemässen Transportsystems ist ferner dadurch gekennzeichnet, dass der erste Behälter aus Glas ist und dass sein Verschluss ein Gummistopfen oder eine Kunststoffkappe oder eine Siegelfolie ist.

Weitere Vorteile und Eigenschaften der vorliegenden Erfindung gehen aus der nachstehenden Beschreibung eines Ausführungsbeispiels hervor, das anhand der beiliegenden Zeichnungen erläutert wird.

Es zeigen

Fig. 1    einen ersten Behälter 11 eines erfindungsgemässen Transportsystems,

Fig. 2    einen zweiten Behälter 14 eines erfindungsgemässen Transportsystems und ein in diesem Behälter enthaltenes Stäbchen 16 mit einem Abstrichtupfer 17,

Fig. 3    eine vergrösserte Ansicht einer erfindungsgemässen, transportbereiten Anordnung bestehend aus einem ersten Behälter 11 gemäss Fig.1 mit einem darin in einem Medium 13 eingeschobenen Stäbchen 16 mit Abstrichtupfer 17 und einem zweiten, mit einer Verschlusskappe 15 verschlossenen Behälter 14 gemäss Fig. 2.

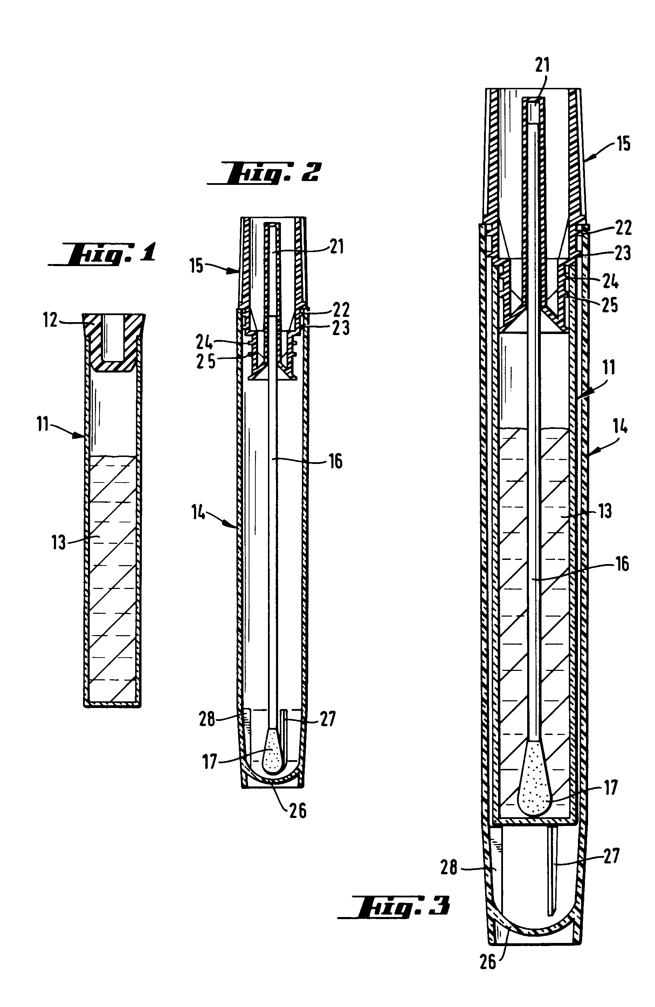

Die Komponenten einer bevorzugten Ausführungsform eines erfindungsgemässen Transportsystems für den Versand einer mikrobiologischen Probe sind in Figuren 1 bzw. 2 in ihrer wirklichen Grösse, d.h. mit dem Masstab 1:1 einzeln dargestellt. Ein solches Transportsystem enthält einen ersten Behälter 11 gemäss Fig. 1 und einen zweiten Behälter 14 gemäss Fig. 2. Der erste Behälter 11 enthält ein geignetes Medium 13 zur Aufbewahrung der Probe und ist mit einem abnehmbaren Verschluss 12 versehen. Der zweite Behälter 14, ist vorzugsweise aus einem geigneten Kunststoff und ist mit einer abnehmbaren Verschlusskappe 15 versehen. Diese Kappe hat eine längliche Kammer 21, in die ein Ende eines Stäbchens 16 angeordnet ist und hineingeschoben werden kann. Das Stäbchen 16 trägt an dessen andererem Ende einen Abstrichtupfer 17. Die Form und die Abmessungen der Behälter 11 und 14, der Bohrung 21, der Verschlusskappe 15 und des Stäbchens 16 mit dem Abstrichtupfer 17 werden so gewählt, dass der erste Behälter 11 und ein darin im Medium 13 eingeschobenes Stäbchen 16 mit Abstrichtupfer 17 in dem mit der Verschlusskappe 15 verschlossenen zweiten Behälter 14 enthalten sein kann.

Die z.Zt. verfügbare Medien 13 haben eine sehr beschränkte Fähigkeit Sauerstoff zu binden. Wenn der zweite Behälter 14 gas-durchlässig ist, ist es deshalb notwendig einen ersten Behälter 11 aus einem gas-undurchlässigen Material zu verwenden, um zu verhindern, dass Sauerstoff zu dem vom Abstrichtupfer getragenen Keim gelangen kann. Der erste Behälter 11 ist daher vorzugsweise aus Glas. Zur Herstellung des ersten Behälters (11) ist jedoch prinzipiell jedes andere gas-undurchlässige Material geignet. Er kann z.B. auch aus einem gas-undurchlässigen Kunststoff, z.B. Copolymer Acryldinitril, hergestellt werden. Hierfür kommen auch andere gas-undurchlässige Materialien wie Metall, Keramik, etc., die jedoch den Nachteil hätten, dass sie undurchsichtig sind.

Wenn der zweite Behälter 14 aus einem gas-undurchlässigen Material hergestellt wird, kann der erste Behälter aus einem gas-durchlässigen Material z.B. einem gas-durchlässigen Kunststoff hergestellt werden.

Steht ein Medium 13 zur Verfügung, der eine so grosse Fähigkeit hat, Sauerstoff zu binden, dass er allein die Probe vom Sauerstoff aus der umgebenden Luft abschirmen kann, dann können sowohl der erste Behälter 11 wie auch der zweite Behälter 14 aus einem gas-durchlässigem Material hergestellt werden.

Die Abmessungen des ersten Behälters 11 gemäss Fig. 1 sind z.B.: Aussendurchmesser: 15 mm, Innendurchmesser: 13 mm, Wandstärke: 1 mm., Länge 100 mm.

In den ersten Behälter 11 werden z.B. 8 ml eines geigneten Mediums abgefüllt, so dass die Füllhöhe des Mediums 13 im ersten Behälter ca. 60 mm beträgt.

Das Medium 13 hat vorzugsweise folgende Zusammensetzung:

| | |
|---|---|
| NaCl | 5 g/l |
| $KH_2PO_4$ | 2 g/l |
| Na-Thioglycolat | 1 g/l |
| L-Cystein HCl | 0,5 g/l |
| Resazurin | 0,002 g/l |
| Kartoffelstärke | 5 g/l |
| Agar-Agar | 8 g/l |

Als Medium 13 sind ausserdem z.B. folgende, an sich bekannten Medien geeignet:

- Transportmedien nach Amies mit und ohne Aktivkohle (Amies, C.R., A modified formula of the preparation of Stuart's Transport Medium", Canadian Journal of Public Health, 58, 296 (1967),

- Transportmedien nach Stuart mit und ohne Aktivkohle (Journal of Pathology and Bacteriology, 58, 343 (1946),
- Transportmedien nach Cary-Blair (Cary, S.G. and Blair, E.B., "New Transport Medium for Shipment of Clinical Specimens. I. Fecal Specimens", Journal of Bacteriology, 88, 96 (1964),.

Wie in der Fig. 1 gezeigt, ist vor der Benutzung des Transportsystems der erste Behälter 11 z.B. mit einem Gummistopfen 12 verschlossen. Anstelle des Gummistopfens 12 könnte auch eine Kunstoffkappe mit oder ohne Schraubverschluss oder eine Siegelfolie verwendet werden. Der erste Behälter ist ausserdem mit einer nicht gezeigten, transparenten Etikette versehen, die ausser dem Namen des Systems, Name und Anschrift des Herstellers, Lagerungshinweis, Verfalldatum und Chargenbezeichnung etc., ein Feld für Patientendaten trägt.

Der zweite Behälter 14 ist vorzugsweise aus glasklaren Polystyrol. Zur Herstellung des zweiten Behälters sind z.B. auch Polyethylen oder Polypropylen geeignet.

Der Durchmesser des zweiten Behälters 14 ist vorzugsweise so zu wählen, dass der erste Behälter 11 problemlos in den zweiten Behälter 14 einge- schoben werden kann und die Wandungen beider Behälter bis auf einen engen Spalt aneinander anliegen. Die Abmessungen des zweiten Behälters 14 gemäss Fig. 2 sind z.B.: Aussendurchmesser: 19 mm, Innendurchmesser: 16 mm, Wandstärke 1,5 mm., Länge 129 mm.

Wie aus Fig. 2 ersichtlich, hat das untere Ende des zweiten Behälters 14 die Form eines Kegelstumpfs. Dieser untere Teil des zweiten Behälters hat einen zurückgesetzten Rundboden 26 und drei Abstandsrippen. Zwei dieser Rippen 27, 28 sind in Fig. 2 dargestellt.

Die Verschlusskappe 15 ist z.B. aus eingefärbtem Polyäthylen hat ein leicht konisches Griffstück mit einer Riffelung und zwei Dichtlippen 22, 23, mit deren Hilfe die Kappe im zweiten Behälter 14 bei dessen Verschluss geklemmt wird. Die Abmessungen des Griffstücks 15 sind z.B.: Länge: 25 mm, oberer Durchmesser: 17 mm, unterer Durchmesser: 18 mm. Am unteren Ende der Kappe befindet sich ein Zylinder, dessen Aussendurchmesser von 13 mm mit dem Innendurchmesser des ersten Behälters übereinstimmt. Dieser Zylinder trägt ebenfalls zwei Dichtlippen 24, 25 und eine längliche Kammer 21 (siehe Fig. 2), in der das Stäbchen 16 des Abstrichtupfers 17 gesteckt ist. Das Stäbchen 16 hat z.B. einen Durchmesser von 2 mm und eine Länge von 125 mm, wobei das Stäbchen 16 soweit in die Kappe 15 gesteckt ist, dass beide zusammen etwa die Länge von gängigen Abstrichtupfern, nämlich 150 mm, besitzen.

Das Stäbchen 16 ist vorzugsweise aus Polypropylen. Zur Herstellung des Stäbchens 16 ist ausserdem Holz oder jeder strahlensterilisierbare Kunststoff geeignet, der sich zu Stäbchen mit hinreichender Steifigkeit ausziehen lässt. Das Watte-Material des Abstrichtupfers 17 kann z.B. aus fettsäurefreier Baumwolle, Zellstoff, Calcium-Alginat, Polyester oder Polyterephthalat bestehen.

Zum bestimmungsgemässen Gebrauch wird der Abstrichtupfer 17 durch Abziehen der Kappe 15 aus dem zweiten Behälter 14 gezogen, am Patienten der Abstrich gemacht und dann der Abstrichtupfer 17 mit der zu transportierenden Probe in den ersten Behälter 11 und in das Medium 13 geschoben, vorzugsweise bis die Spitze des Abstrichtupfers 17 am Boden des ersten Behälters 11 anstösst, damit der ganze Abstrichtupfer 17 auf jeden Fall vom Medium 13 umgeben wird. Beim Einschieben des Abstrichtupfers 17 in das Medium 13 gleitet das obere Ende des Stäbchens 16 des Abstrichtupfers 17 in die Kammer 21 der Kappe 15 und die Kappe verschliesst die Oeffnung des ersten Behälters mit Hilfe ihres unteren, inneren Zylinders. Der erste Behälter 11 mit dem Abstrichtupfer 17 und die Kappe 15 werden danach in den zweiten Behälter 14 gesteckt, wodurch die in Fig. 3 gezeigte Anordnung gebildet wird. Wie in Fig. 3 gezeigt, werden für den Transport der Probe der erste Behälter 11 und der zweite Behälter 14 durch die Verschlusskappe 15 verschlossen. Dabei klemmt die Kappe 15 mit Hilfe der äusseren zwei Dichtlippen 22, 23 den ersten Behälter 11 in dem zweiten Behälter 14. Ein unerwünschtes Oeffnen des ersten Behälters während des Transportes durch Abfallen des ersten Behälters 11 von der Kappe 15 wird durch die Unterstützung des ersten Behälters 11 durch die drei Abstandsrippen im unteren Teil des zweiten Behälters 14 verhindert. Das so gebildete Transportsystem schützt den ersten Behälter 11 gegen Bruch und verhindert auf diese Weise ein durch Bruch dieses Behälters verursachtes Auslaufen dessen halbfesten Inhaltes.

Wie aus der Fig. 3 ersichtlich, sind die Abmessungen des ersten Behälters 11 und des zweiten Behälters 14, der Kammer 21, der Verschlusskappe 15 und des Stäbchens 16 mit dem Abstrichtupfer 17 so gewählt, das der erste Behälter 11 und einen darin im Medium 13 eingeschobenen Stäbchen 16 mit Abstrichtupfer 17 in dem mit der Verschlusskappe 15 verschlossenen zweiten Behälter 14 enthalten sind.

Die Verschlusskappe 15 ist vorzugsweise so gestaltet, dass sie gemäss Fig. 3 sowohl der erste als auch der zweite Behälter gleichzeitig verschliessen kann.

Wie aus den Figuren 2 und 3 ersichtlich, ist ein Ende des Stäbchens 16 vor der Benutzung des Abstrichtupfers 17 (Fig.2) und nach dessen Einführung im Medium 13 unterschiedlich weit in die Kammer 21 der Verschlusskappe 15 eingeschoben.

Wie aus Fig. 3 ersichtlich, hat die Kammer 21 der Verschlusskappe 15 vorzugsweise einen Durchmesser, der in der Richtung vom offenen Ende der Kappe zum geschlossenen Ende derselben leicht abnimmt.

**Patentansprüche**

1. Transportsystem für den Versand von biologischen Proben, dadurch gekennzeichnet, dass es folgende Komponenten enthält:

   (a) einen ersten Behälter (11), der mit einem abnehmbaren Verschluss (12) versehen ist und ein Medium (13) zur Aufbewahrung der Probe enthält, und

   (b) einen zweiten Behälter (14), der mit einer abnehmbaren Verschlusskappe (15) versehen ist, die eine längliche Kammer (21) hat, in die ein Ende eines Stäbchens (16) angeordnet ist und hineingeschoben werden kann, welches Stäbchen an dessen andererem Ende einen Abstrichtupfer (17) trägt,

   wobei die Abmessungen des ersten und des zweiten Behälters, der Kammer (21) in der Verschlusskappe (15) und des Stäbchens (16) mit dem Abstrichtupfer (17) so gewählt werden, dass der erste Behälter (11) und einen darin im Medium (13) eingeschobenes Stäbchen (16) mit Abstrichtupfer (17) in dem mit der Verschlusskappe (15) verschlossenen zweiten Behälter (14) enthalten sein können.

2. Transportsystem gemäss Anspruch 1, dadurch gekennzeichnet, dass die Verschlusskappe (15) des zweiten Behälters (14) so gestaltet ist, dass wenn der verschlossene zweite Behälter den ersten Behälter (11) und ein darin im Medium (13) eingeschobenes Stäbchen (16) mit Abstrichtupfer (17) enthält, die Verschlusskappe (15) gleichzeitig den ersten und den zweiten Behälter verschliesst.

3. Transportsystem gemäss Anspruch 1, dadurch gekennzeichnet, dass die Kammer (21) annähernd zylindrisch ist und einen Durchmesser hat, der in der Richtung vom offenen Ende der Kappe zum geschlossenen Ende derselben leicht abnimmt.

4. Transportsystem gemäss Anspruch 1, dadurch gekennzeichnet, dass der Verschluss (12) des ersten Behälters (11) ein Gummistopfen oder eine Kunststoffkappe oder eine Siegelfolie ist.

5. Transportsystem gemäss Anspruch 1, dadurch gekennzeichnet, dass der erste Behälter (11) aus einem gas-undurchlässigem Material und der zweite Behälter (14) aus einem gas-durchlässigem Material ist.

6. Transportsystem gemäss Anspruch 5, dadurch gekennzeichnet, dass der erste Behälter (11) aus Glas und der zweite Behälter (14) aus einem gas-durchlässigem Kunststoff ist.

7. Transportsystem gemäss Anspruch 1, dadurch gekennzeichnet, dass der erste Behälter (11) aus einem gas-durchlässigem Material und der zweite Behälter (14) aus einem gas-undurchlässigem Material ist.

8. Transportsystem gemäss Anspruch 1, dadurch gekennzeichnet, dass der erste Behälter (11) und der zweite Behälter (14) aus je einem gas-durchlässigem Material sind, und das Medium (13) eine so hohe Kapazität zur Bindung von Sauerstoff aufweist, dass wenn der Abstrichtupfer (17) in das Medium (13) eingeschoben ist, das Medium (13) eine vom Abstrichtupfer (17) getragene Probe vom Sauerstoff abschirmt, der in der Umgebungsluft enthalten ist.

Fig. 1

Fig. 2

Fig. 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-4 150 950   (TAKEGUCHI) <br> * Spalte 2, Zeile 18 - Seite 43 *** Spalte 2, Zeile 54 - Spalte 3, Zeile 19; Abbildungen 1-3 ** <br> – – – | 1,2 | G 01 N 1/02 <br> C 12 M 1/30 <br> A 61 B 10/00 |
| Y | US-A-4 915 255   (CURTIS) <br> * Spalte 1, Zeile 51 - Zeile 65 *** Spalte 2, Zeile 51 - Spalte 3, Zeile 5; Ansprüche 3,4; Abbildungen 2,8 ** <br> – – – | 1,2 | |
| A | CH-A-330 179   (BÖTTGER) <br> * Seite 1, Zeile 45 - Zeile 64 *** Seite 2, Zeile 69 - Seite 3, Zeile 42; Abbildung 2 ** <br> – – – | 1 | |
| A | EP-A-0 328 932   (BECTON DICKINSON) <br> * Zusammenfassung; Ansprüche 1,2; Abbildung 1 ** <br> – – – – – | 1,4-6 | |

| RECHERCHIERTE SACHGEBIETE (Int. Cl.5) |
|---|
| G 01 N <br> C 12 M <br> A 61 B <br> B 01 L <br> A 61 F |

Der vorliegende Recherchenbericht wurde für alle Patentansprüche erstellt

| Recherchenort | Abschlußdatum der Recherche | Prüfer |
|---|---|---|
| Den Haag | 16 Oktober 91 | MILLS J. |